# EUROPEAN PATENT SPECIFICATION

(11) **EP 2 062 573 B1**
(45) Date of publication and mention of the grant of the patent: **16.11.2011**
(21) Application number: 08020810.1
(22) Date of filing: 30.04.2002
(51) Int. Cl.: A61K 9/70, A61M 35/00, A61F 13/00

(54) **Abuse resistant opioid containing transdermal systems**
Missbrauchssichere Opioide mit transdermalen Systemen
Opioïde résistante aux abus contenant des systèmes transdermiques

(30) Priority: 01.05.2001 US 287875 P; 22.05.2001 US 292537 P
(43) Date of publication of application: 27.05.2009
(62) Divisional of application: 02731653.8
(73) Proprietor: EURO-CELTIQUE S.A., 1653 Luxembourg (LU)
(72) Inventor: Tavares, Lino, Kinnelon, NJ 07405 (US); Reidenberg, Bruce E., Rye, NY 10580 (US); Sackler, Richard S., Greenwich, CT 06830 (US); Wright, Curtis, Norwalk, CT 06851 (US); Alfonso, Mark A., Purcell, Oklahoma 73080 (US); Oshlack, Benjamin, Boca Raton, FL 33432 (US); Cassidy, James P., Cortlandt Manor,NY 10567 (US); Carpanzano, Anthony E., Sherman, CT 06784 (US); Gullapalli, Rampurna Prasad, Elmsford, NY 10523 (US); Shevchuk, Ihor, Yonkers, NY 10710 (US)
(74) Representative: Maiwald, Walter

(56) References cited:
- WO-A-97/04835
- WO-A-99/32120
- WO-A-02/085268
- US-A- 5 149 538
- US-A- 5 236 714

## Description

### FIELD OF THE INVENTION

This invention relates to a tamper-resistant article and to a transdermal dosage form that prevents misuse of the medicament therein. The dosage form includes at least one inactivating agent that is released when the article or dosage form is misused. Preferably, the inactivating agent is a crosslinking agent that crosslinks or degrades the medicament contained within the article or patch. However, the inactivating agent is not delivered to a patient by the transdermal route. In addition, the article or patch may further include an antagonist, *e.g.,* an opiate antagonist, to reduce abuse of the medicament in the dosage form.

### BACKGROUND OF THE INVENTION

Transdermal dosage forms are convenient dosage forms for delivering many different active therapeutically effective agents, including but not limited to analgesics, such as for example, opioid analgesics. Typical opioid analgesics include, but are not limited to, fentanyl, buprenorphine, etorphines, and other high potency narcotics. Typical non-opioid drugs which are delivered by the transdermal route are anti-emetics (scopolamine), cardiovascular agents (nitrates & clonidine) and hormones (estrogen & testosterone).

The most common transdermal dosage form is a diffusion-driven transdermal system (transdermal patch) using either a fluid reservoir or a drug-in-adhesive matrix system. Other transdermal dosage forms include, but are not limited to, topical gels, lotions, ointments, transmucosal systems and devices, and iontohoretic (electrical diffusion) delivery system.

Transdermal dosage forms are particularly useful for timed release and sustained release of active agents. However, many dosage forms, and particularly those for timed and sustained release of active agent(s), contain large amounts of active agent(s), often many times the actual absorbed dose. Often, the dosage form contains an excess of active agent or delivers less than the entire amount of its active agent to the subject being treated. This results in some of the active agent remaining in the dosage form after use. Both the unused dosage form and the portion of active agent that remains in the dosage form after use are subject to potential illicit abuse, particularly if the active agent is a narcotic or a controlled substance. For example, used dosage forms containing excess or unused opioids may be tampered with by chewing or extraction by a drug abuser. Even careful disposal of used dosage forms may not be completely effective in preventing abuse, particularly in cases of incomplete or partial compliance.

U.S. Patent No. 5,149,538 to Granger et al. ("Granger") relates to a misuse-resistive dosage form for the transdermal delivery of opioids.

WO 97/04835 describes a transdermal system for administering a substance through the skin, the system including a reservoir containing the substance to be administered as an agonist as well as an associated antagonist. The system also includes at least one electrode which is polarized, when the transdermal system is used, so that only the agonist passes into the skin.

US 5,236,714 relates to compositions and dosage forms for administering abuseable substances. The compositions and dosage forms are said to have a reduced potential for abuse without diminishing the therapeutic or beneficial effects of the abuseable substance.

WO 99/32120 relates to a method of reducing the abuse potential of an oral dosage form of an opioid analgesic, wherein an analgesically effective amount of an orally effective opioid agonist is combined with an opioid antagonist into an oral dosage form which would require at least a two-step extraction process to be separated from the opioid agonist, wherein the amount of included opioid antagonist is sufficient to counteract opioid effects if extracted together with the opioid agonist and administered parenterally.

There is a need for a transdermal dosage form that is less susceptible to abuse by misuse of the active agent remaining in an unused dosage unit or in a used dosage unit, *i.e.,* residual active agent

### BRIEF DESCRIPTION OF DRAWINGS

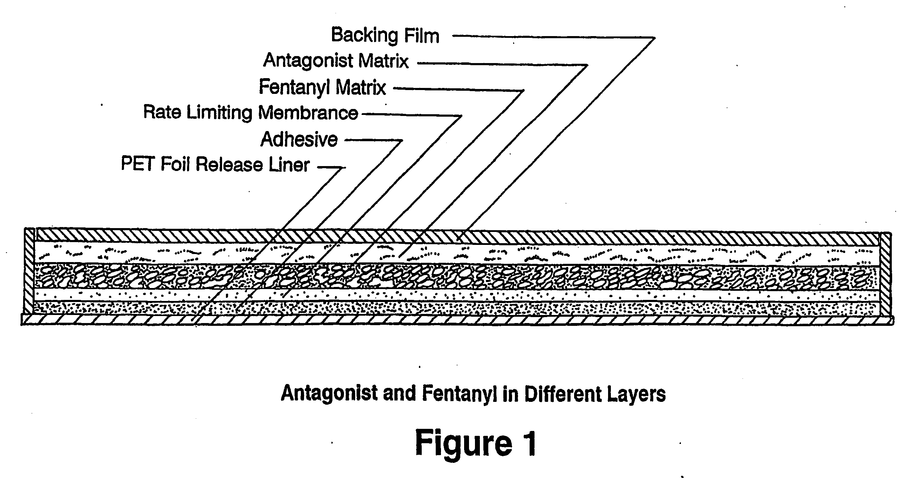

Figure 1. A schematic of a Fentanyl transdermal system design where the antagonist and Fentanyl are in separate layers.

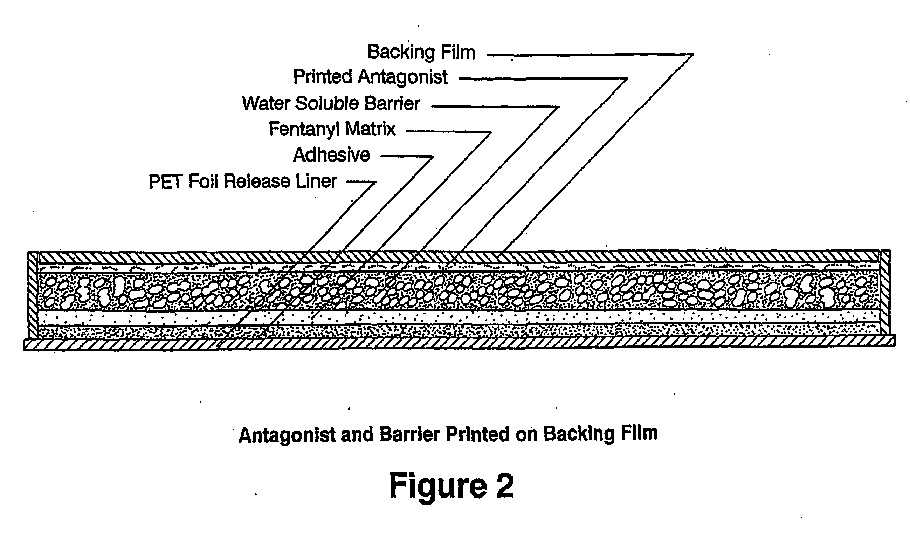

Figure 2. A schematic of a Fentanyl transdermal system design where the antagonist and barrier are printed on backing film.

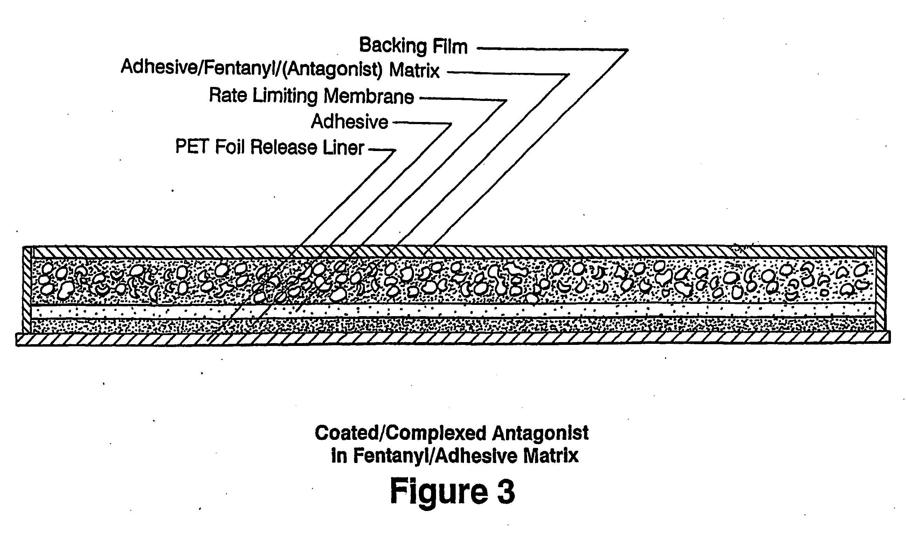

Figure 3. A schematic of a Fentanyl transdermal system design where the coated/complexed antagonist is present in a Fentanyl/adhesive matrix.

### SUMMARY OF THE INVENTION

It is an object of the present invention to provide a transdermal dosage article, *e.g*., a transdermal patch, wherein the dosage form includes at least one activating agent and at least one inactivating agent. The inactivating agent is released only when the dosage form is solubilized, opened, chewed and/or cut apart, but it is not transdermally delivered to the patient. Therefore, the article or composition prevents or hinders misuse of the active agent contained in the transdermal dosage form. In a preferred embodiment, the inactivating agent is a crosslinking agent. In a further embodiment, the crosslinking agent is selected from the group consisting of polymerizing agent, photoinitiator, and aldehydes (*e.g*., formalin). Preferably, the polymerizing agent is selected from the group consisting of diisocyanate, organic peroxide, diimide, diol, triol, epoxide, cyanoacrylate, and a UV activated monomer. In an additional embodiment, the dosage form further comprises an antagonist, preferably an opiate antagonist.

The present specification describes a transdermal dosage article or a transdermal dosage composition comprising (1) a matrix comprising at least one active agent and (2) beads comprising at least one inactivating agent, wherein the beads are mixed into the matrix. The beads may further comprise an antagonist. In a specific embodiment, the beads are comprised of microscopic polysaccharide beads, starch beads, polyactate beads, or liposomes. In a further embodiment, the beads are dissolved upon contact with an aqueous solvent or a non-aqueous solvent.

The present specification describes a transdermal dosage article or a transdermal dosage composition comprising (1) a matrix comprising at least one active agent and (2) a polymer complexed to at least one inactivating agent. In a further embodiment, the polymer also may be complexed to at least one antagonist. In a specific embodiment, the polymer is a crosslinked styrene divinyl benzene polymer. The polymer may optionally be linked to a solid support, such as a resin. In a further embodiment, the inactivating agent and the antagonist uncomplex from the polymer in an ionic solvent

The present invention contemplates a transdermal dosage article comprising (1) a first layer comprising at least one active agent, (2) an inactivating layer comprising at least one inactivating agent, and (3) a solvent soluble membrane or solvent soluble layer. In a further embodiment, the inactivating layer further comprises an antagonist In a specific embodiment, the solvent soluble membrane or solvent soluble layer is comprised of hydroxy ethyl cellulose and hydroxypropylmethylcellulose. In a further embodiment, the solvent is water.

### DETAILED DESCRIPTION

The present invention provides a transdermal dosage article, *e.g*., a transdermal patch , wherein the dosage form includes at least one inactivating agent. The dosage form may further comprise at least one antagonist The inactivating agent and the antagonist may be present in different portions of the dosage article or may be present in a combination. The combination of the inactivating agent and antagonist may be included in any part of the transdermal dosage article, such as in an adhesive coating present in a transdermal patch. The inactivating agent and/or antagonist are released only when the dosage article is solubilized, opened, chewed or cut apart. The inactivating agent and the antagonist are sequestered within the transdermal dosage article and are not transdermally delivered to the patient. Therefore, during storage and regular use, the inactivating agent and antagonist are not in direct contact or are not in sufficient contact with the activating agent to render the active agent inactive. However, if the dosage article is misused for the purpose of being abused, such as for example, is chewed, soaked, subjected to extraction, smoked, or the like, the inactivating agent and/or the antagonist are released. The inactivating agent would degrade or cross-link the medicament or active agent to decrease its efficacy. Additionally, the antagonist would block the effects produced by the activating agent. The present invention further discloses methods of preparing such abuse resistant dosage articles.

Transdermal dosage forms may be classified into transdermal dosage articles and transdermal dosage compositions. The most common transdermal dosage article is a diffusion-driven transdermal system (transdermal patch) using either a fluid reservoir or a drug-in-adhesive matrix system. Transdermal dosage compositions include, but are not limited to, topical gels, lotions, ointments, transmucosal systems and devices, and ionthoretic (electrical diffusion) delivery system. Preferably, the transdermal dosage form is a transdermal patch. The dosage article contains both at least one active agent and at least one first inactivating agent

As used herein, the terms "active agent" or "activating agent" refer to a compound that produces a pharmacological effect that leads to a physiological change. Examples of active agents which may be used in the present invention are fentanyl, buprenorphine, etorphine and related opioids of sufficient potency to allow transdermal usage, or any combinations thereof. Non-opioid drugs that may be used include, anti-emetics (scopolamine), cardiovascular agents (nitrates & clonidine) and hormones (estrogen & testosterone). In a specific embodiment of the present application, the active agent is an opioid analgesic used in the treatment of pain. Most preferably, the active agent is fentanyl.

As used herein, the term "antagonist" refers to a compound that renders the active agent unavailable to produce a pharmacological effect. In other words, the antagonist, itself, does not produce a pharmacological effect, but rather blocks the effects of an active agent. In a specific embodiment, the antagonist interacts with the same receptor as the active agent and inhibits the interaction of the active agent with the receptor. Non-limiting examples of antagonists include opioid-neutralizing antibodies; narcotic antagonists such as naloxone, naltrexone and nalmefene; dysphoric or irritating agents such as scopolamine, ketamine, atropine or mustard oils; or any combinations thereof. In a preferred embodiment, the antagonist is naloxone or naltrexone.

As used herein, the term "inactivating agent" refers to a compound that inactivates or crosslinks the medicament, in order to decrease the abuse potential of the transdermal dosage form. Non-limiting examples of a inactivating agents include polymerizing agents, photoinitiators, and formalin. Examples of polymerizing agents include diisocyanabes, peroxides, diimides, diols, triols, epoxides, cyanoacrylates, and UV activated monomers.

### Transdermal Dosage Systems

Any dosage article that is capable of sequestering an inactivating agent and allows for the release of these agents under abuse conditions is contemplated by the present specification. These systems may further comprise an antagonist. Such transdermal dosage systems include, but are not limited to, solvent-based systems, ion-concentration based systems, and barrier systems.

A solvent-based system can be used to release the inactivating agent only under contact with specific solvents. Such solvents include, but are not limited to, a salt solution, ether, dimethylfuran, alcohol, chloroform, acetone, benzene, dimethylformamide, methylene chloride, toluene, formaldehyde, ethyl acetate, celusolve; cosmetic agents such as, nail polish remover and glycerine; paint/printing agents such as, methyl ethyl ketone, mineral spirits, turpentine; fuels such as, gasoline and kerosene; dry cleaning fluids; or an aqueous solvent similar to saliva. The solvent-based system is comprised of microscopic beads that are porous to the solvents. The beads that can be used in the invention include, but are not limited to, microscopic polysaccharide beads; starch beads; polylactate beads; polylactategluconate beads (PLGA);beads made from mineral based microporous catalyst materials such as zeolite, alumina, hydroxyapetite, and silica; beads made from hydrogels such as polyethyleneoxide, polyacrylamide, and polyvinylpyrolidone; beads made from chromatography media such as ion exchange resins, size exclusion beads, and affinity resins; beads made from natural source gels such as chitosan, gelatin, celluloses, and agarose; and beads made from sintered porous supports/filters such as brass, aluminum, and glass. In a specific embodiment, the beads are microscopic polysaccharide beads; starch beads; or polyactate beads. The solvent-based system may also use liposomes. The release of the inactivating agent is dependent upon the chemical properties of the bead or liposome used in the present invention. For example, when starch beads are used, the inactivating agent is released when contacted with an aqueous environment.

An ion-concentration based system can be used to preferentially release the inactivating agent when contacted with an ionic solvent. In such a system, the agent would be complexed or bound to an exchange resin present in the dosage form. The resin can be of any form known in the art. The exchange resin may be comprised of at least one resin selected from the group consisting of styrene divinylbenzene, an acrylic matirix with an anion and/or cation functional groups, and a silica matrix with anion and/or cation functional groups. Preferably the resin is a crosslinked styrene divinyl benzene polymer or a cation exchange resin, *e.g.* Amberlite IR-122. Functional groups may include, but are not limited to, R-CH₂N⁺(CH₃)₃; R-CH₂N⁺(CH₃)₂C₂H₄OH; R-SO₃-; R-CH₂N⁺H(CH₃)₂; R-CH₂COO-; R-COO-; and R-CH₂N(CH₂COO)₂. When the dosage form is contacted with an ionic solvent, such as an alcohol or water, the agent would be released. In one embodiment, the solvent contains ethanol and water. In a specific embodiment, the ion concentration of the solvent needed to release the first inactivating agent is about 100 mEq/liter. To further ensure dissociation in the saliva, the resin can be further constructed to be cleaved by salivary enzymes such as, but not limited to, salivary amylase.

A layered based system comprises a dosage form in which the active agent and the inactivating agent are present in different layers of the dosage form. For example, the active agent is in a first matrix layer and the inactivating agent is in an inactivating matrix layer of the dosage form or vice versa. The layers may be separated by a barrier, such as a solvent soluble layer or a solvent soluble membrane. The barrier may be soluble in ether, dimethylfuran, alcohol, chloroform, acetone, benzene, dimethylformamide, methylene chloride, toluene, formaldehyde, ethyl acetate, celusolve; cosmetic agents such as, nail polish remover and glycerine; paint/printing agents such as, methyl ethyl ketone, mineral spirits, turpentine; fuels such as, gasoline and kerosene; dry cleaning fluids; or an aqueous solvent similar to saliva. In a specific embodiment, the barrier is soluble in water, alcohol, ether, chloroform, and dimethylfuran. The barrier may be comprised of any material known in the art, such as cellulose film. The barrier may take the form of an adhesive layer, such as, but not limited to, a (hydro)gel layer, polymer based film, woven or non-woven support, porous sponge material, dispersed coated particles or beads. Additionally, the barrier can be protected by a poly(ethylene terephthlate) (PET) release liner. Dosage forms wherein the inactivating agent is in a reservoir also are contemplated by the present invention. As previously discussed, an antagonist also may be present in the inactivating layer.

A transdermal patch produced according to the present specification may be produced in three different forms. In one embodiment, a matrix comprising the inactivating agent is coated onto a backing film. The active agent is then coated onto this matrix. This dosage form then optionally comprises a barrier, an adhesive layer, and a PET release liner. In another embodiment, the inactivating agent is coated onto a backing film. This layer is then separated from a matrix comprising the active agent by a water soluble layer. This dosage form then optionally comprises an adhesive layer and a PET release liner. In another embodiment, a matrix comprising the active agent and the inactivating agent is coated onto a backing film. This matrix may optionally comprise an adhesive. This dosage form may further comprise a barrier, an adhesive, and a PET release liner.

### Methods for Preparing Transdermal Dosage Systems

The inactivating agent and antagonist may be incorporated in the transdermal dosage form by any methods that are known in the art. Listed herein are a few of the preferred methods for incorporating the first inactivating agent into the dosage form. Additionally discussed are the proposed methods of how the formulations would prevent abuse of the dosage form.

### Layer Based System

In this system, a layer of at least one inactivating agent is applied to one side of the transdermal dosage article. The layer may further comprise an antagonist. This layer than can be separated from the active agent by a solvent soluble layer or a solvent permeable microporous membrane. In a specific embodiment, the solvent is water. The water soluble layer may be comprised of any substance that may be soluble in a specific solvent, such as hydroxy ethyl cellulose and hydroxypropylmethylcellulose. The layer of the inactivating agent may be applied to the transdermal dosage form by any method known in the art. In one embodiment, the inactivating agent can be applied to the dosage form by 3-dimensional printing technology. Once the inactivating layer is placed on the dosage form, the soluble membrane or layer is placed in contact with it The active agent then may be applied, by any method to the soluble membrane or layer. When the dosage form is placed into an aqueous solvent, the soluble membrane or layer may dissolve and the inactivating agent is released.

### Administration

The dosage articles of the present invention may be used treat various conditions depending on the medicament contained within the dosage form. In a preferred embodiment, the medicament is used to treat pain. The dosage articles of the present invention may be administered alone at appropriate dosages defined by routine testing in order to obtain optimal activity while minimizing any potential toxicity.

The amount of active agent in a dosage form will depend upon the needs of the patient and the characteristics of the patient such as, for example, height, weight, age, and gender. Such amounts can be determined by those skilled in the art by methods such as establishing a matrix of amounts and effects. However, such amounts should be those amounts effective to achieve the results sought through the dosage article. For example, the amount of an opioid analgesic active agent in such a dosage article should be that amount effective to deliver analgesia to a patient for the amount of time for which the dosage article is to be used.

The amounts of the inactivating active agent and antagonist will depend upon the active agent and the amount of residual active agent that is expected in a particular dosage article. Such amounts can also be determined by those skilled in the art by methods such as establishing a matrix of amounts and effects. However, such amounts should be those amounts effective to achieve the results sought, *i.e*., inactivation of the residual active agent or the rendering of undesirable of an attractive drug of abuse.

### Testing Methods for Transdermal Articles

Any method may be used to assess the tamper-resistance of the present invention. In a specific embodiment, the active agent will be extracted from the transdermal article with a solvent. Such solvents include, but are not limited to, a salt solution, ether, dimethylfuran, alcohol, chloroform, acetone, benzene, dimethylformamide, methylene chloride, toluene, formaldehyde, ethyl acetate, celusolve; cosmetic agents such as, nail polish remover and glycerine; paint/printing agents such as, methyl ethyl ketone, mineral spirits, turpentine; fuels such as, gasoline and kerosene; dry cleaning fluids; or an aqueous solvent similar to saliva. After extraction, the active agent present in the solvent and in the article will be assessed using any method known in the art. Such methods include, measurement with infrared or ultraviolet/visible spectrophotometry, chromatography techniques such as high performance liquid chromatography or gas chromatography. The technique should both identify and quantify the active agent in the phosphate buffer and in the composition or article.

In another specific embodiment, the transdermal article will be mechanically separated. A small animal surgeon will be provided the schematics of the test transdermal article. The surgeon, using operating room tools, will then dissect the patch to separate the material containing the active agent from the inactivating agent. The surgeon may be provided with test patches for practice. The test may be timed. After separation, chemical analysis will be performed on the separated materials to determine the degree of success. Any method known in the art may be used for chemical analysis. Such methods include, measurement with infrared or ultraviolet/visible spectrophotometry, chromatography techniques such as high performance liquid chromatography or gas chromatography.

### EXAMPLES

The present invention will be better understood by reference to the following Examples, which are provided as exemplary of the invention, and not by way of limitation.

### Reference EXAMPLE I: EXTRACTION IN ETHANOL CONTAINING SOLVENT

The transdermal patch is macerated with 100 mL of a solvent containing 75% ethanol. The patch is macerated for 30 minutes. The drugs that are present in the solvent and in the patch are measured by high performance liquid chromatography.

### Reference EXAMPLE II: EXTRACTION IN ETHER CONTAINING SOLVENT

The transdermal patch is macerated with 100 mL of a solvent containing reagent grade diethyl ether. The patch is macerated for 30 minutes. The drugs that are present in the solvent and in the patch are measured by high performance liquid chromatography.

### Reference EXAMPLE III: MECHANICAL SEPARATION

A small animal surgeon is provided the schematics of the test transdermal dosage form. The surgeon, using operating room tools, dissects the patch to separate the material containing the active agent from the inactivating agent. The surgeon is provided with two test patches for practice. The time limit for the actual experiment is 1 hour. Chemical analysis is performed on the separated materials to determine the degree of success. The drugs that are present are measured by high performance liquid chromatography.

### Reference EXAMPLE IV: EXTRACTION OF AGENTS IN AOUEOUS SOLVENT TO DUPLICATE SALIVA

A transdermal patch is prepared, wherein the patch comprises an active agent and inactivating agent. The patch is placed in a roller bottle, with the adhesive side facing the solvent. The solvent is a 0.5 N NaCl solution that is buffered to pH = 6.4 with a phosphate buffer. About 15 mL of the solvent is placed in the bottle and the bottle then is rolled at 20 rpm for 30 minutes. The drugs that are present in the solvent and in the patch are measured by high performance liquid chromatography.

## Claims

1. A transdermal dosage article for preventing misuse of the medicament therein comprising a first layer comprising at least one active agent, an inactivating layer comprising at least one inactivating agent, and a solvent soluble membrane or solvent soluble layer.

2. A transdermal dosage article of claim 1, wherein the solvent soluble membrane or solvent soluble layer is comprised of hydroxy ethyl cellulose and hydroxypropylmethylcellulose.

3. A transdermal dosage article of claim 1, wherein the solvent is water.

4. The transdermal dosage article of claim 1 wherein the inactivating agent is formalin.

5. The transdermal dosage article of claim 1 wherein the active agent is fentanyl, buprenorphine, etorphine, scopolamine, a nitrate, clonidine, estrogen or testosterone.

## Patentansprüche

1. Transdermaler Dosierungsgegenstand zum Verhindern des Missbrauchs eines darin enthaltenen Medikaments, umfassend eine erste Schicht, welche mindestens einen Wirkstoff umfasst, eine Inaktivierungsschicht, welche mindestens ein Inaktivierungsmittel umfasst, und eine in einem Lösemittel lösliche Membran oder in einem Lösemittel lösliche Schicht.

2. Transdermaler Dosierungsgegenstand gemäß Anspruch 1, wobei die in einem Lösemittel lösliche Membran oder die in einem Lösemittel lösliche Schicht Hydroxyethylcellulose und Hydroxypropylmethylcellulose umfasst.

3. Transdermaler Dosierungsgegenstand gemäß Anspruch 1, wobei das Lösemittel Wasser ist.

4. Transdermaler Dosierungsgegenstand gemäß Anspruch 1, wobei das Inaktivierungsmittel Formalin ist.

5. Transdermaler Dosierungsgegenstand gemäß Anspruch 1, wobei der Wirkstoff Fentanyl, Buprenorphin, Etorphin, Scopolamin, ein Nitrat, Clonidin, Östrogen oder Testosteron ist.

## Revendications

1. Article de dosage transdermique destiné à empêcher la mauvaise utilisation du médicament contenu à l'intérieur comprenant une première couche comprenant au moins un agent actif, une couche d'inactivation comprenant au moins un agent d'inactivation et une membrane soluble dans un solvant ou une couche soluble dans un solvant.

2. Article de dosage transdermique selon la revendication 1, dans lequel la membrane soluble dans un solvant ou la couche soluble dans un solvant est composée d'hydroxyéthylcellulose et d'hydroxypropylméthylcellulose.

3. Article de dosage transdermique selon la revendication 1, dans lequel le solvant est l'eau.

4. Article de dosage transdermique selon la revendication 1, dans lequel l'agent d'inactivation est une solution acqueuse de formaldéhyde.

5. Article de dosage transdermique selon la revendication 1, dans lequel l'agent actif est le fentanyl, la buprénorphine, l'étorphine, la scopolamine, un nitrate, la clonidine, l'oestrogène ou la testostérone.
